# EUROPEAN PATENT SPECIFICATION

(11) **EP 4 106 745 B1**
(45) Date of publication and mention of the grant of the patent: **20.03.2024**
(21) Application number: 21708575.2
(22) Date of filing: 18.02.2021
(51) Int. Cl.: A61K 31/353, A61P 15/02, A61K 31/355, A61K 9/48, A61K 9/00

(54) **CAPSULE COMPRISING A VITAMIN E ESTER FOR PREVENTING THE ONSET OF VAGINAL DYSBIOSIS AND RESULTING VAGINAL PATHOLOGIES**
KAPSEL ENTHALTEND EINEN VITAMIN E-ESTER ZUR PRÄVENTION DER ENTWICKLUNG VON VAGINALER DYSBIOSE UND DARAUS RESULTIERENDEN VAGINALEN ERKRANKUNGEN
CAPSULE COMPRENANT UN ESTER DE LA VITAMINE E POUR LA PREVENTION DU DEVELOPPEMENT DE LA DYSBIOSE VAGINALE ET DES PATHOLOGIES VAGINALES QUI EN RESULTENT

(30) Priority: 21.02.2020 IT 202000003620
(43) Date of publication of application: 28.12.2022
(73) Proprietor: HULKA S.R.L., 45100 Rovigo (IT)
(72) Inventor: PANIN, Giorgio, 45100 Rovigo (IT)
(74) Representative: Botti & Ferrari S.p.A.
(86) International application number: PCT/EP2021/054035
(87) International publication number: WO 2021/165402

(56) References cited:
- WO-A1-00/02554

## Description

### Field of Application

The present invention refers to the field of pharmaceutical industry.

In particular, the present invention relates to a formulation in the form of a capsule for vaginal application, in which the content of said capsule consists of a vitamin E acetate or vitamin E n-propionate, as further defined in the claims, for use in preventing the onset of vaginal dysbiosis and resulting vaginal pathologies.

### Prior art

Vitamin E and derivatives thereof are substances that are widely used in the pharmaceutical and cosmetic industry, in the preparation of formulations for treating skin diseases or for counteracting or preventing skin blemishes, thanks to their antioxidant and free radical-scavenging properties.

Application WO 00/02554 describes the use of vitamin E and in particular of tocopherol acetate for the treatment of mucosa diseases, including pathologies of the vaginal mucosa, such as atrophic vaginitis and vaginal dryness and itchiness, by virtue of tocopherol acetate's emollient, protective and lubricant properties.

However, it is not mentioned any effect of vitamin E or esters thereof on the vaginal bacterial flora.

Nevertheless, it is well known that the vaginal flora of healthy women mainly consists of lactobacilli (or Döderlein bacilli), which regulate the growth of the remaining bacterial flora and impede vaginal colonization by pathogenic germs. These functions are due to the ability of Döderlein bacilli to hinder adhesion of other microorganisms to the vaginal mucosa, to synthesize hydrogen peroxide, which has a bactericidal effect, and to acidify the vaginal environment (which has a pH of 4-4.5 in fertile age and 6-7 in postmenopausal age), metabolizing glycogen and producing lactic acid.

In addition to lactobacilli, the vaginal bacterial flora comprises to a minor extent also streptococci, enterobacteria, anaerobic microorganisms, gardnerella, candida and mycoplasmas. Some of these microorganisms, even though they are potentially pathogenic, are not able to exert harmful effects, since they are kept in a limited number by Döderlein bacilli and by the immune system.

However, in some instances the vaginal flora may be altered and in this case the balance of vaginal microorganisms, or homeostasis of the vaginal ecosystem, is broken and the growth of pathogenic microorganisms may increase, resulting in pathologies of the vaginal mucosa. This may happen, for example, during the intake of antibiotics and immunosuppressive drugs, or as a consequence of diabetes or psychophysical stress.

In order to restore homeostasis of the vaginal ecosystem, one may use lactic acid-based products, to bring the pH back to about 4-4.5 and thus facilitate the growth of Döderlein bacilli, and preparations containing lactobacilli, as well as antibiotics specific for certain microorganisms.

"Döderlein bacilli" means in the present application the lactobacilli population characteristic of the vaginal environment, which comprises as predominant species *Lactobacillus acidophilus* together with many other species also belonging to the *Lactobacillus* genus, including *Lactobacillus fermentum, Lactobacillus plantarum, Lactobacillus brevis, Lactobacillus jensenii, Lactobacillus casei, Lactobacillus cellobiosus, Lactobacillus leichmanii, Lactobacillus delbrueckii, Lactobacillus salivarius.*

In its research, the Applicant has investigated the possible interactions between Döderlein bacilli and vitamin E-based products, in particular alpha-tocopheryl acetate. In particular, the Applicant verified if the vaginal capsules marketed as Filme Gyno and Filme Gyno-V from Panin S.r.l. had an effect on the growth of Döderlein bacilli.

The performed experimental investigations surprisingly showed that said capsules containing alpha-tocopheryl acetate improve the growth ability of Döderlein bacilli, thus causing the maintenance of the homeostasis of the vaginal ecosystem.

### Summary of the invention

An object of the present invention is thus a formulation in the form of a capsule for vaginal application, in which the content of the capsule consists of a vitamin E ester selected from the group consisting of vitamin E acetate and vitamin E n-propionate, for use in preventing the onset of vaginal dysbiosis and resulting vaginal pathologies, wherein said capsule comprises an envelope consisting of the following ingredients: hydroxypropyl starch, glycerol, iota carrageenan, water and dibasic sodium phosphate and has the following composition in percentage by weight of the dry weight of the envelope:

| | |
|---|---|
| hydroxypropyl starch | 40-52% |
| glycerol | 32-41% |
| iota carrageenan | 12-18% |
| dibasic sodium phosphate | 1.0-1.8% |

Vitamin E may be used in all its forms (tocopherols and tocotrienols, their isomers alpha, beta, gamma, delta) and derivatives.

Preferably, the ester is alpha-tocopherol acetate.

Preferably, each capsule contains 300-700 mg, conveniently about 500 mg, of alpha-tocopheryl acetate.

Preferably, the envelope of each capsule has a weight of 180-275 mg, preferably 225-250 mg.

To maintain homeostasis of the vaginal ecosystem, it is sufficient to administer by vaginal route one capsule per day. The administration may be conveniently performed in the evening before going to bed.

Among the pathologies of the vaginal mucosa that can be prevented by the use of the formulation according to the invention, the following are cited: bacterial vaginosis, in particular bacterial vaginosis associated with Gardnerella, nonspecific vaginitis, Candida vulvovaginitis, vaginal yeast infections and chlamydia.

The vaginal capsules for the use according to the present invention may be produced by known methods. For instance, reference can be made to G. Reich, "Formulation and physical properties of soft capsules", chapter 11, 2004, and WO 01/03677.

The present invention will be further described with reference to some examples.

### Brief description of the figures

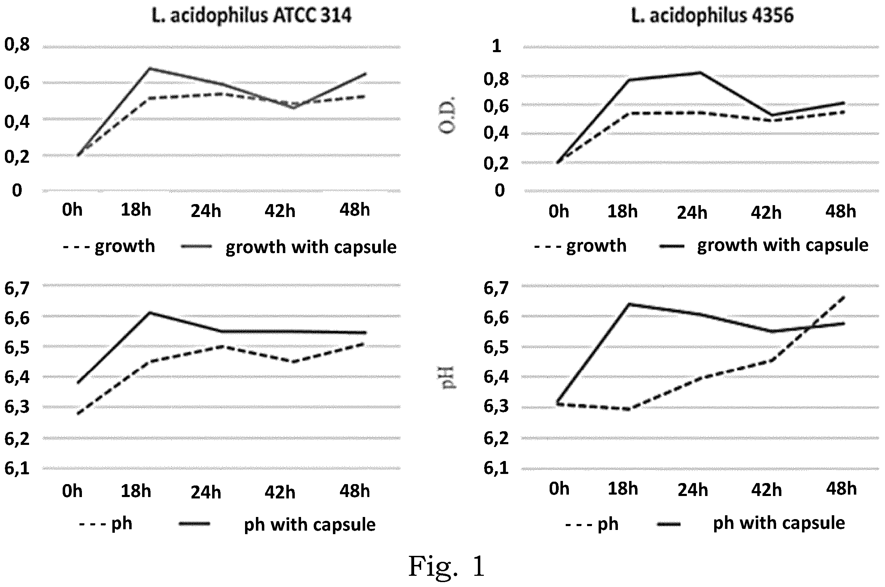

Figure 1 consists of four diagrams showing the density and the pH, measured at time zero and after 18, 24, 42 and 48 hours, of broth cultures of Lactobacillus acidophilus ATCC 314 (on the left) and of Lacobacillus acidophilus ATCC 4356 (on the right) which were supplemented with the product Filme Gyno-V (solid lines) and the corresponding control broth cultures (no addition of the product Filme Gyno-V - dashed lines).

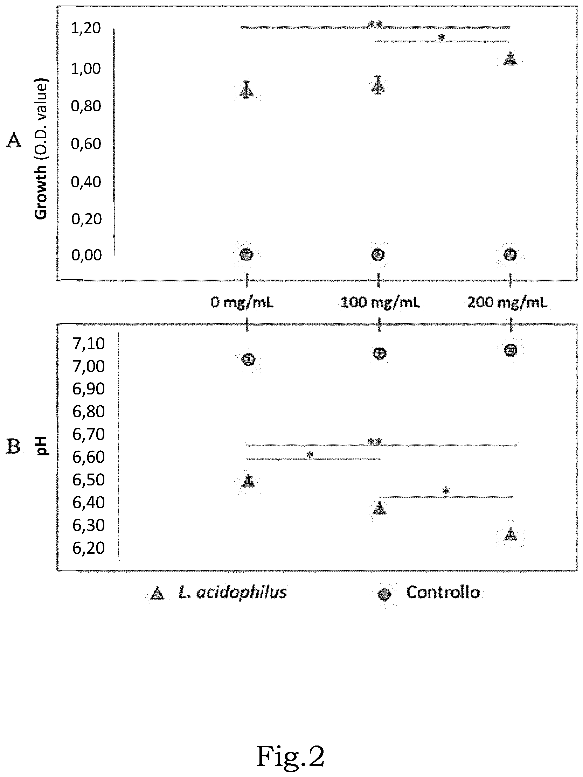

Figure 2 consists of two diagrams A and B, which respectively show the optical density and the pH of cultures of *Lactobacillus acidophilus* ATCC 4356, measured in the presence of the components of the envelope of the Filme Gyno-V capsules at concentrations of 100 and 200 mg/ml and in the absence of said components.

### Detailed description

### EXAMPLE 1

An in vitro study was performed in order to verify the effect of the Filme Gyno-V capsules on the growth of two standard strains of *Lactobacillus acidophilus* (ATCC 314 and ATCC 4356).

Each Filme Gyno-V capsule contains 500 mg alpha-tocopheryl acetate and has an envelope of about 250 mg weight and which has the following composition, in percentages by weight of the total dry weight of the envelope:

| | |
|---|---|
| hydroxypropyl starch | 46.92% |
| glycerol | 36.52% |
| iota carrageenan | 15.18% |
| dibasic sodium phosphate | 1.38% |

Aim of this study was to verify the potential interference of the product Filme Gyno-V with the bacteria of the human vaginal microbiota (Döderlein bacillus) responsible for the physiological maintenance of a low vaginal pH.

In particular, it was sought to verify if the whole capsules could influence the growth of lactobacilli and/or cause at the same time pH variations. The conditions are interdependent and contribute to the homeostasis of the vaginal ecosystem. The loss of this homeostatic mechanism is an important factor which could lead to the onset of a state of vaginal dysbiosis (CRIBBY, Sarah; TAYLOR, Michelle; REID, Gregor. *Vaginal microbiota and the use of probiotics. Interdisciplinary perspectives on infectious diseases,* 2008).

For this purpose, a possible in vitro interference of the product Filme Gyno-V with the growth of two standard strains of *Lactobacillus acidophilus* (ATCC 314 and ATCC 4356) was tested. Contextually, the pH in the culture medium in the presence of the product was also monitored at different times.

### Materials and methods

Two series of 4 broth cultures for each lactobacillus were prepared in duplicate. The broth cultures were produced by inoculating the microorganisms grown overnight so as to obtain an initial theoretical optical density (O.D). equal to 0.2 O.D.

The broth cultures were produced in medium *"Brain Heart Infusion Broth"* (Oxoid) or "BHI" or "Brain Heart Infusion") (Code CM 1135) having the following composition:

| | |
|---|---|
| Brain infusion solids | 12.5 g/l |
| Beef heart infusion solids | 5.0 g/l |
| Peptocomplex (Proteose peptone) | 10.0 g/l |
| Glucose | 2.0 g/l |
| Sodium chloride | 5.0 g/l |
| Dibasic sodium phosphate | 2.5 g/l |

The vaginal capsules were added to one of the two series of 4 broth cultures (one capsule for each broth culture), while the other series of broth cultures was used as a control of the growth in the absence of Filme Gyno-V. All the broth cultures were incubated at 37°C and maintained under slow agitation (100 rpm) to facilitate complete and homogeneous dissolution of the capsule in the culture medium. After incubation of 18, 24, 42 and 48 hours, the optical density (O.D.) of the respective broth cultures was measured in order to estimate the microbial growth that had taken place.

Moreover, two additional series of 4 broth cultures for each lactobacillus were prepared in duplicate. The broth cultures were produced by inoculating the microorganisms grown overnight so as to obtain an initial theoretical optical density (O.D) equal to 0.2 O.D.

The broth cultures were produced in medium LSM (Klare et al., 2005; doi:10.1128/AEM.71.12.8982-8986.2005), an unbuffered medium.

The vaginal capsules were added to one of the two series of 4 broth cultures (one capsule for each broth culture), while the other series of broth cultures was used as a control of the pH variation in the absence of Filme Gyno-V. All the broth cultures were incubated at 37°C and maintained under slow agitation (100 rpm) to facilitate complete and homogeneous dissolution of the capsule in the culture medium. After incubation of 18, 24, 42 and 48 hours, the pH of the respective broth cultures was measured.

Finally, the growth and pH data recorded in the presence of the vaginal capsule were compared to those obtained in the absence of the same (CAMPBELL, Jennifer. High-throughput assessment of bacterial growth inhibition by optical density measurements. Current protocols in chemical biology, 2010, 2.4: 195-208).

### Results

The results of this study are summarized in Figure 1.

As it is clear from said figure, the addition of Filme Gyno-V to the culture medium improves the growth ability of both the lactobacilli; said effect is more pronounced for the strain ATCC 4356 which maintains an increased growth rate in the presence of the capsule at all measurement times.

The increase in growth rate in the presence of the capsule is already evident during the log growth phase (18-24 hours) for both strains; this could represent the time required for the components of the capsule to completely dissolve in the medium.

For the strain ATCC 314, the highest increase in microbial growth is recorded after an 18-hour incubation and it is equal to an increase of 24% with respect to the control condition, while for the strain ATCC 4356 the maximum increase of 34% is obtained after a 24-hour incubation.

As regards the pH variations in the presence of the capsule, variation trends opposite to the control are recorded. For both strains, in the absence of the capsule, starting from 18 hours of incubation the pH value gradually increases, while in the presence of the capsule the pH values tend to gradually decrease and said effect is particularly evident between 18 and 24 hours of incubation for the strain ATCC 314 and for an even longer time for the strain ATCC 4356, for which the pH reduction is recorded from 18 to 42 hours of incubation.

### EXAMPLE 2

The effect of only the components of the envelope of Filme Gyno-V capsules on the growth of Döderlein bacilli and on the ability of the same to acidify the growth medium was also evaluated.

### Materials and methods

For this purpose, capsules with a weight of 100 mg were produced, consisting only of the components of the envelope of Filme Gyno-V capsules, according to the proportions of said components in the marketed product:

| | |
|---|---|
| hydroxypropyl starch | 46.92% |
| glycerol | 36.52% |
| iota carrageenan | 15.18% |
| dibasic sodium phosphate | 1.38% |

The above percentages are by weight of the total dry weight of the envelope.

Cultures of *Lactobacillus acidophilus* ATCC 4356 were prepared in triplicate together with triplicates of only medium BHI as controls of the experiment, both in a final volume of 1.5 ml.

*Lactobacillus acidophilus* ATCC 4356 was grown overnight at 37°C; 50 µl of the overnight-grown culture were inoculated in a final volume of 1.5 ml of medium BHI to produce fresh cultures of the microorganism. Controls consisting of 1.5 ml of only medium BHI without bacterial inoculum were prepared.

Both the controls and the L. acidophilus cultures were inoculated, each one in triplicate and respectively with no capsule, one or two capsules.

The cultures and the controls were maintained in incubation for 24 hours, the time required for complete capsule dissolution.

After said incubation time, two parameters were measured, pH variation and microbial growth. The dose-response effect of the components of the envelope of the capsule was evaluated both in the controls and in the cultures of the microorganism.

### Results

In the absence of lactobacillus (control) increasing concentrations of the components of the envelope of the capsule (100 and 200 mg/ml) do not influence the pH of the culture medium, which remains at approximately neutrality values (6.9-7.1) (Fig. 2B).

When the components of the envelope are added to the culture medium of lactobacillus, a clear influence on the growth of the microorganism and on the pH is recorded (Fig. 2). As known, the growth of the lactobacillus is accompanied by a significant reduction of the pH of the surrounding medium which, from values of 7.0 measured in the absence of bacterial growth (for the control), reaches values of 6.50 in the presence of only the bacterial inoculum.

By adding to the bacterial culture the components of the envelope at a concentration of 100 mg/ml (1 capsule), a further pH reduction to the value of 6.40 occurs (Fig. 2B), accompanied by an increase of the microorganism growth compared to the control culture (in the absence of the capsule) (Fig. 2A). However, the data obtained about the growth in these conditions are not statistically significant.

Vice versa, the pH variation induced by the presence of the components of the capsule was statistically significant compared to the control conditions.

This let us hypothesize that, at these concentrations, the components of the envelope influence the metabolism of the microorganism rather than its growth, wherein said metabolism is directed to a larger production of lactic acid compared to the metabolism that occurs in the absence of the components of the envelope.

By adding instead the components of the envelope at a concentration of 200 mg/ml (2 capsules), a dual effect is observed: both the microbial growth rate and the tendency to acidification of the culture medium are significantly increased, the culture medium reaching in this case pH values of 6.30, as compared to the value of 6.50 obtained in the absence of the components of the envelope and with only the growth of the microorganism.

The above results suggest that the Filme Gyno-V vaginal capsules induce an increase of the growth rate of lactobacilli in a range between 20 and 30 % and modifications of the pH of the growth milieu of said microorganisms with the tendency to acidification of the same.

The effect is the result of the combined action of alpha-tocopheryl acetate included in the Filme Gyno-V capsules and of the components of the envelope of said capsules. Also the latter showed their action towards Döderlein bacillus. Compared to the capsule considered as a whole, the components of the envelope alone stimulate more mildly the growth of the microorganism but influence even at low concentrations the metabolism of the same, directing it to a higher production of lactic acid. Indeed, the pH of the growth medium in the presence of the components of the envelope decreases according to a dose-response effect.

From the above experimental results it is evident that alpha-tocopheryl acetate significantly enhances the growth of Döderlein bacilli and causes a significant acidification of the milieu in which said bacilli grow. It can be expected that such advantageous effects of alpha-tocopheryl acetate and, in general, of vitamin E and esters thereof, occur also in the vaginal milieu, thus contributing to the maintenance of homeostasis of the vaginal ecosystem and to the prevention of any vaginal dysbiosis.

It is also noted that the envelope of the capsules containing alpha-tocopheryl acetate exerts an advantageous action on Döderlein bacilli, in synergy with the action exerted by alpha-tocopheryl acetate.

## Claims

1. A formulation in the form of a capsule for vaginal application, in which the content of said capsule consists of a vitamin E ester selected from the group consisting of vitamin E acetate and vitamin E n-propionate, for use in preventing the onset of vaginal dysbiosis and resulting vaginal pathologies, wherein said capsule comprises an envelope consisting of the following ingredients: hydroxypropyl starch, glycerol, iota carrageenan, water and dibasic sodium phosphate and has the following composition in percentage by weight of the dry weight of the envelope:
| | |
|---|---|
| hydroxypropyl starch | 40-52% |
| glycerol | 32-41% |
| iota carrageenan | 12-18% |
| dibasic sodium phosphate | 1.0-1.8% |

2. The formulation for the use according to claim 1, wherein said vitamin E ester is alpha-tocopheryl acetate.

3. The formulation for the use according to claim 2, wherein said capsule contains 300-700 mg, preferably about 500 mg of alpha-tocopheryl acetate.

4. The formulation for the use according to claim 3 wherein said envelope has a weight of 180-275 mg.

## Patentansprüche

1. Formulierung in Form einer Kapsel zur vaginalen Anwendung, in der der Inhalt der Kapsel aus einem Vitamin-E-Ester besteht, ausgewählt aus der Gruppe bestehend aus Vitamin E-Acetat und Vitamin E-n-Propionat, zur Verwendung bei der Prävention des Auftretens von vaginaler Dysbiose und resultierenden vaginalen Erkrankungen, wobei die Kapsel eine Umhüllung umfasst, die aus den folgenden Bestandteilen besteht: Hydroxypropylstärke, Glycerin, Iota Carrageen, Wasser und zweibasisches Natriumphosphat und weist die folgende Zusammensetzung in Gewichtsprozent des Trockengewichts der Umhüllung auf :
| | |
|---|---|
| Hydroxypropyl-Stärke | 40-52% |
| Glycerin | 32-41 % |
| Iota Carrageen | 12-18% |
| Zweibasisches Natriumphosphat | 1.0-1.8% |

2. Formulierung für die Verwendung gemäß Anspruch 1, wobei der Vitamin E-Ester Alpha-Tocopherylacetat ist.

3. Formulierung für die Verwendung gemäß Anspruch 2, wobei die Kapsel 300-700 mg, vorzugsweise etwa 500 mg Alpha-Tocopherylacetat enthält.

4. Formulierung für die Verwendung gemäß Anspruch 3, wobei die Umhüllung ein Gewicht von 180-275 mg aufweist.

## Revendications

1. Formulation dans la forme d'une capsule pour application vaginale, dans laquelle la teneur de ladite capsule consiste en un ester de vitamine E choisi dans le groupe consistant en acétate de vitamine E et n-propionate de vitamine E, pour une utilisation dans la prévention de l'apparition de dysbiose vaginale et pathologies vaginales résultantes, dans laquelle ladite capsule comprend une enveloppe consistant en les ingrédients suivants : hydroxypropylamidon, glycérol, carraghénane iota, eau et phosphate de sodium dibasique et présente la composition suivante en pourcentage en masse de la masse sèche de l'enveloppe :
| | |
|---|---|
| hydroxypropylamidon | 40-52 % |
| glycérol | 32-41 % |
| carraghénane iota | 12-18 % |
| phosphate de sodium dibasique | 1,0-1,8 % |

2. Formulation pour l'utilisation selon la revendication 1, dans laquelle ledit ester de vitamine E est l'acétate d'alpha-tocophéryle.

3. Formulation pour l'utilisation selon la revendication 2, dans laquelle ladite capsule contient 300-700 mg, de préférence environ 500 mg d'acétate d'alpha-tocophéryle.

4. Formulation pour l'utilisation selon la revendication 3, dans laquelle ladite enveloppe présente une masse de 180-275 mg.
